# EUROPÄISCHE PATENTSCHRIFT

(11) **EP 3 000 419 B1**
(45) Veröffentlichungstag und Bekanntmachung des Hinweises auf die Patenterteilung: **07.09.2016**
(21) Anmeldenummer: 14186623.6
(22) Anmeldetag: 26.09.2014
(51) Int. Cl.: A61B 17/22, A61B 17/225, A61H 23/00

(54) **Gerät zur Behandlung des menschlichen oder tierischen Körpers mit mechanischen Stößen**
Device for treatment of the human or animal body with mechanical impacts
Appareil à chocs mécaniques pour le traitement du corps humain ou animal

(43) Veröffentlichungstag der Anmeldung: 30.03.2016
(73) Patentinhaber: STORZ MEDICAL AG, 8274 Tägerwilen (CH)
(72) Erfinder: Novak, Pavel, 8234 Stetten (CH); Schulz, Johannes Manfred, 8274 Tägerwilen (CH); Swart, Stephan Gerhard, 47447 Moers (DE); Di Maio, Carlo, 47199 Duisburg (DE); Piontkowski, Ulrich, 74321 Bietighelm-Bissingen (DE)
(74) Vertreter: König Szynka Tilmann von Renesse Patentanwälte Partnerschaft mbB

(56) Entgegenhaltungen:
- DE-A1- 19 725 477
- DE-A1-102004 042 895
- DE-U1-202014 004 070
- US-A- 4 498 464
- US-A- 4 549 535
- US-A1- 2002 177 795
- US-A1- 2009 221 940

## Beschreibung

Die vorliegende Erfindung bezieht sich auf ein Gerät zur Behandlung des menschlichen oder tierischen Körpers durch Ausüben von Stößen auf die Körperoberfläche. Im Folgenden wird zur Vereinfachung vom Körper eines Patienten gesprochen, der bevorzugt menschlich ist.

Im Stand der Technik sind verschiedene Geräte des beschriebenen Grundtyps bekannt. Die DE 197 25 477 C beschreibt bspw. ein solches Gerät, bei dem durch die Kollision eines pneumatisch beschleunigten Schlagteils oder Projektils mit einem zunächst ruhenden Prallkörper oder Applikator eine Druckwelle ausgelöst wird, die dadurch in den Körper des Patienten eingekoppelt werden kann, dass eine Applikatorfrontfläche zum Zeitpunkt der Kollision auf den Patientenkörper aufgelegt wird. Dieser Gerätetyp leitet sich in seiner Entstehungsgeschichte von Lithotripsiegeräten ab, bei denen solche Druckwellen z. B. über eine lange stabförmige Sonde an der Frontfläche des Applikators auf einen Nierenstein oder Ähnliches übertragen werden können, um diesen zu desintegrieren.

Der Schwerpunkt bei der Darstellung liegt in jedem Fall auf der durch die Kollision erzeugten Druckwelle, die mehr oder weniger einer eigentlichen Stoßwelle ähneln soll, wie sie klassische, in der Regel fokussierende Lithotripsiegeräte z. B. mit piezoelektrischen oder induktiven Aktuatoren und Fokussierung auf einen Stein erzeugen. Solche Druckwellen können Anstiegsflanken mit einer Breite im Bereich weniger µs und einer Amplitude im niedrigen zweistelligen MPa-Bereich haben (z. B. 2 µs und 15 MPa gemessen 1 cm vor der Frontfläche). In dem zitierten Dokument wird hingegen betont, dass die physikalisch an sich unvermeidliche makroskopische Schwerpunktbewegung des Applikators möglichst klein gehalten werden soll, weil sie als störend angesehen wird.

Als zweites Beispiel wird auf die DE 20 2004 011 323 U und, mit sehr ähnlichem Inhalt, die US 2011/0054367 A1 verwiesen. Dort wird ein hinsichtlich des technischen Aufbaus ähnliches Gerät beschrieben, bei dem allerdings u. a. die elastische Aufhängung des Applikators im Gehäuse auf größere Schwerpunktsbewegungen des Applikators ("Hübe") ausgelegt ist. Dort wird betont, dass die therapeutische Wirkung durchaus auch, oder vorwiegend in den eigentlichen makroskopischen Stößen (also infolge des Hubes) gesehen werden kann, was auch von der Indikation abhängt.

Die vorliegende Erfindung richtet sich allgemein auf Geräte dieses Bautyps, und zwar sowohl hinsichtlich der Anwendung von Druckwellen als auch hinsichtlich der Anwendung "makroskopischer Stöße" des Applikator auf die Körperoberfläche.

Zum Stand der Technik wird verwiesen auf die US 2002/177795 A1, die ein pneumatisch betriebenes chiropraktisches Stoßgerät gemäß dem Oberbegriff des Anspruchs 1 beschreibt, dessen Applikator nach vorne eine Kappe mit einer stumpf-konischen Frontfläche trägt. Ferner wird verwiesen auf die DE 20 2014 004 070 U1, in der in Figur 6 ein Applikator mit zwei schrägen Frontflächen dargestellt ist. Ferner wird verwiesen auf die US 4,498,464 A und die US 2009/221940 A1.

Dabei liegt der Erfindung die Aufgabe zugrunde, ein solches Gerät hinsichtlich weitergehender Anwendungsmöglichkeiten auf der Patientenkörperoberfläche weiterzubilden.

Die Aufgabe wird gelöst durch ein Gerät nach Anspruch 1. Erfindungsgemäß ist dabei zunächst vorgesehen, dass der Applikator einen schrägen Frontflächenanteil hat, der zumindest 30 % einer in Bezug auf die Stoßrichtung vorderen Frontfläche des Applikators beträgt, der in Bezug auf die Stoßrichtung schräg nach außen weist und der dabei einen Winkel zu der Stoßrichtung zwischen 30° und 60° bildet.

Dementsprechend ist also eine Schrägfläche als wesentlicher Bestandteil der Frontfläche des Applikators vorgesehen. "Schräg" bedeutet dabei einen Winkel zu der Stoßrichtung zwischen 30° und 60°, wobei die Stoßrichtung in vielen praktisch wichtigen Fällen (z. B. bei Geräten in einer Bauform entsprechend zitierten Dokumenten) einer Längsrichtung des Geräts entspricht.

Die "Frontfläche" des Applikators soll die nach vorne weisenden Teile der Außenfläche beinhalten, die auf die Patientenkörperoberfläche aufgesetzt werden können, wobei "nach vorne weisend" bedeutet, dass eine lokale Flächennormale eine Komponente in Richtung der Stoßrichtung (und nicht etwa entgegengesetzt dazu oder nur senkrecht dazu) hat. Zur Stoßrichtung parallel liegende Flächen gehören also nicht zur Frontfläche und nach hinten weisende auch nicht.

Zusätzlich sind der schräge Frontflächenanteil und vorzugsweise die gesamte Frontfläche erfindungsgemäß kantenfrei. Das soll konkret bedeuten, dass die auftretenden Krümmungsradien mindestens 2 mm betragen, vorzugsweise mindestens 3 mm, 4 mm oder sogar mindestens 5 mm. Auch das kommt einem Einsatz des Geräts unter Krafteinwirkung durch den Therapeuten und/oder mit einer Schiebebewegung entgegen; der Therapeut muss dabei weniger auf die Haltung des Geräts und/oder auf mögliche Reizungen oder Verletzungen des Patienten durch Kanten achten, etwa Kanten am Rand einer eigentlich aufzusetzenden Applikatorfläche.

Schließlich soll die Schrägfläche insoweit einen wesentlichen Anteil der Frontfläche ausmachen, als sie zumindest 30 % ihres Flächenanteils beträgt, wobei ein Anteil von (in der folgenden Reihenfolge zunehmend bevorzugt) 35 %, 40 %, 45 %, 50 %, 55 %, 60 %, 65 %, 70 % zunehmend bevorzugt ist. Die Flächen werden dabei berücksichtigt, wie sie tatsächlich dreidimensional existieren, also nicht etwa als Projektionsflächen mit Blickrichtung von vorn.

Die Erfinder haben nämlich festgestellt, dass es praktisch sein kann, den Applikator in Bezug auf die Stoßrichtung schräg auf die Patientenkörperoberfläche aufzusetzen. Das betrifft insbesondere Behandlungen, bei denen das Gerät auf der Körperoberfläche bewegt wird. Dann greift sich das Gerät besser und kann in einer für den Therapeuten besser kontrollierbaren und bequemeren Weise über die Körperoberfläche geführt werden. Insbesondere können dabei Gewebepartien unter der Haut parallel massiert und/oder gewissermaßen vor dem Gerät hergeschoben werden, wobei sie gleichzeitig mit den Stößen und (in einem von Fall zu Fall unterschiedlichen Maß) Druckwellen beaufschlagt werden. Die konventionell bekannten Geräte mit mehr oder weniger flachen Frontflächen der Applikatoren sind dazu relativ schlecht geeignet, weil sie auf der Hautoberfläche nur bei mehr oder weniger senkrechter Stellung des Geräts relativ zur Körperoberfläche gehalten werden können.

Der Mechanismus zum Erzeugen der Stöße des Applikators ist in diesem Zusammenhang in unterschiedlichster Ausführung denkbar, insbesondere auch durch eine direkte Beaufschlagung des Applikators mittels eines elektromagnetischen Mechanismus oder auch eines Druckfluidpulses Bevorzugt ist aber die aus den zitierten Dokumenten bereits bekannte Verwendung eines beschleunigten Projektils zur Kollision mit dem Applikator, womit relativ heftige und (im Sinn der Applikatorgeschwindigkeit) schnelle Stöße realisiert werden können und auch die gleichzeitige oder sogar schwerpunktmäßige Verwendung von Druckwellen möglich ist. Das Projektil wiederum lässt sich ebenfalls in unterschiedlicher Weise beschleunigen, insbesondere auch elektromagnetisch, wobei auch hier eine pneumatische Beaufschlagung des Projektils gemäß den zitierten Schriften bevorzugt ist.

Der Begriff "Applikator" bezeichnet hier übrigens nicht zwingend ein einstückiges Teil. Es ist denkbar und anwendungsabhängig auch bevorzugt, dass das Projektil z. B. auf ein erstes Teil schlägt, das den Stoß und/oder die Druckwelle auf ein zweites Applikatorteil überträgt, das seinerseits mit der Haut des Patienten in Kontakt steht. Im Stand der Technik ist auch gelegentlich von einem Zwischenstück zwischen Applikator und Projektil die Rede; hier wird dann von einem mehrteiligen Applikator gesprochen, wobei die beiden Applikatorteile fest verbunden sein können, aber nicht sein müssen.

Wie eingangs bereits erwähnt, kann ein relativ großer Hub des Applikators durchaus gewünscht sein, wobei im vorliegenden Zusammenhang das Gerät vorzugsweise so ausgelegt ist, dass Hübe über 1 mm erreichbar sind, wie schon in der US 2011/0054367 A1 ausgeführt.

Die beschriebene schräge Fläche (in den Ansprüchen der schräge Frontflächenanteil) ist vorzugsweise nicht konkav, also flach oder konvex (was einen kombiniert flach-konvexen Fall beinhaltet), um einen guten Kontakt mit der Haut bzw. Körperoberfläche und eine konzentrierte Krafteinwirkung beim Auflegen zu ermöglichen.

Ferner ist der schräge Frontflächenanteil insgesamt vorzugsweise mindestens 1,5 cm² groß, wobei in der folgenden Reihenfolge folgende Untergrenzen zunehmend bevorzugt sind: 1,75 cm² und schließlich 2,0 cm². Mit zunehmender Flächengröße lässt sich ein größerer Gewebebereich erfassen und die Kraft des Stoßes und/oder die aufdrückende Kraft des Therapeuten breiter verteilen. Die oben genannten Werte beziehen sich dabei auf die gesamte schräge Frontfläche, wobei anwendungsabhängig nur ein Teil davon genutzt wird, zum Beispiel bei einer im Wesentlichen auf zwei Seiten verteilten Frontfläche nur die Hälfte auf der gerade zum Patienten hin orientierten Seite.

Ferner ist bevorzugt, dass ein großer Anteil des schrägen Frontflächenanteils relativ flach verläuft. Hier geht es nicht um die Kantenfreiheit, sondern um das Ausmaß in der Fläche verbleibender Krümmungen. Mindestens 80 % des Flächenanteils sollten einen Krümmungsradius von mindestens 5 mm aufweisen, vorzugsweise von mindestens 6 oder sogar 7 mm. Bei bestimmten Ausführungsformen mit praktisch flachen schrägen Frontflächenanteilen ist sogar eine Untergrenze von 10 mm, 20 mm, 30 mm, 40 mm oder sogar 50 mm bevorzugt.

Ein weiterer Aspekt der Erfindung betrifft die Symmetrie des Frontflächenanteils relativ zur Stoßrichtung (in der Regel auch Längsrichtung des Geräts). Im Stand der Technik sind, soweit bekannt, grundsätzlich rotationssymmetrische Applikatorgeometrien beschrieben. Im vorliegenden Fall aber sind gerade nicht vollständig rotationssymmetrische Geometrien bevorzugt. Grundsätzlich ist natürlich eine gewisse Symmetrie, also mit einer begrenzten Zähligkeit vorstellbar und, wie die Ausführungsbeispiele zeigen, durchaus auch günstig, wobei jedoch höchstens eine vierfache Zähligkeit, vorzugsweise nur eine dreifache oder sogar zweifache Zähligkeit bevorzugt sind. Anschaulich gesprochen bedeutet das, dass bei einer bestimmten Zähligkeit entsprechend viele einander symmetrisch entsprechende, also durch Drehung ineinander überführbare, schräge Frontflächenanteile vorhanden sind. Bei einer zweizähligen Symmetrie sind also zwei Schrägflächen gegeben, die beide in gleicher Weise eingesetzt werden können, und bei einer Dreizähligkeit derer drei. Eines der Ausführungsbeispiele zeigt eine nicht rotationssymmetrische, in diesem Fall allerdings spiegelsymmetrische Variante.

In einem vergleichbaren Sinn ist ferner bevorzugt, dass sich der schräge Frontflächenanteil gewissermaßen auf einen Teil eines Rotationswinkels um die Stoßrichtung konzentriert. Anschaulich gesprochen bedeutet das, dass die schrägen Frontflächenanteile bei einem Blick auf den Applikator entgegengesetzt zur Stoßrichtung (also von vorn) nicht gleichmäßig um den Applikator herum verteilt sind, sondern auf 4, 3, 2 oder sogar nur eine Schrägfläche konzentriert.

Vorbekannte Applikatoren bestanden im Regelfall aus rostfreiem Stahl. Im vorliegenden Fall kommen grundsätzlich auch Metalle, so auch rostfreier Stahl, in Betracht. Daneben sind als metallische Materialien Aluminium und Titan zu nennen, wobei beide eine relativ geringe Massendichte haben und damit relativ leichte Applikatoren ermöglichen. Das kann den Vorteil haben, bei gegebener Geometrie eine stärkere Beschleunigung und damit auch größere Hubauslenkung des Applikators zu ermöglichen, was im vorliegenden Fall gewünscht sein kann. Titan zeichnet sich zudem durch eine besonders hohe mechanische Belastbarkeit aus, eignet sich also besonders für Anwendungen mit vergleichsweise hohen Projektilgeschwindigkeiten und/oder Projektilmassen. Da Titan außerdem eine besonders gute physiologische Verträglichkeit auszeichnet, kann es als Applikatormaterial auch unabhängig von der Belastbarkeit von Vorteil sein.

In Betracht kommen ferner Keramiken, vgl. Anmeldenummer 08 003 840.9 / EP 2 095 843, und Kunststoffe. Im Vergleich zu rostfreiem Stahl weisen auch diese den Vorteil geringer Massendichte auf. Sie haben ferner eine geringere Wärmeleitfähigkeit als Metalle, so dass der Patient den Applikator subjektiv als wärmer empfindet. Das gilt vor allem für Kunststoffe. Kunststoffe kommen insbesondere auch dann in Betracht, wenn auf die Einkopplung einer Druckwelle geringerer Wert gelegt wird, weil, jedenfalls bei einer größeren Stärke des Kunststoffs, im Vergleich zu den vorgenannten Materialien etwas größere Wellenleitungsverluste zu erwarten sind. Gleiche Argumente gelten für Holz.

Im Folgenden wird die Erfindung auch anhand zweier Ausführungsbeispiele näher erläutert, deren einzelne Merkmale im Rahmen des geltenden Anspruchs 1 auch unabhängig voneinander und in anderen Kombinationen erfindungswesentlich sein können.
- Figur 1: zeigt ein Druckwellengerät als erstes Ausführungsbeispiel der Erfindung mit einem in Fig. 2 näher dargestellten Applikator.
- Figur 2: zeigt den Vorderteil des Applikators des Geräts aus Fig. 1 in gleicher Blickrichtung wie in Fig. 1 (aber aufrecht) in Teildarstellung a, in einer Schnittdarstellung mit um 90° um die Längsachse verdrehter Blickrichtung in Teildarstellung b, in einer weiteren Darstellung c in Längsrichtung des Geräts gesehen und schließlich in Teildarstellung d in perspektivischer Ansicht.
- Figur 3: zeigt ein zweites Ausführungsbeispiel analog Fig. 1, aber mit einem anderen, in Fig. 4 dargestellten Applikator.
- Figur 4: zeigt den Vorderteil des Applikators des Geräts aus Fig. 2 in gleicher Blickrichtung wie in Fig. 1 (aber aufrecht) in Teildarstellung a, in einer Schnittdarstellung mit um 90° um die Längsachse verdrehter Blickrichtung in Teildarstellung b, in einer weiteren Darstellung c in Längsrichtung des Geräts gesehen und schließlich in Teildarstellung in d in perspektivischer Ansicht.

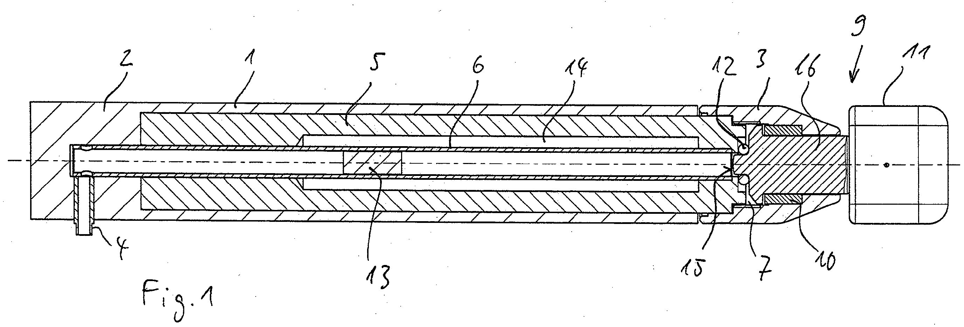

Figur 1 zeigt ein erstes Ausführungsbeispiel der Erfindung. Es handelt sich um ein Gerät zur Einkopplung von Stößen und unfokussierten (so genannten radialen) mechanischen Druckwellen in den menschlichen oder tierischen Körper.

Ein Rohrstück 1 bildet gemeinsam mit einer in der Anwendung körperabgewandten und mit dem Rohrstück 1 integrierten Zuluftkappe 2 und einer in der Anwendung körperzugewandten Applikatorkappe 3 ein Gehäuse. Die Zuluftkappe 2 enthält einen Druckluftanschluss 4 für eine pneumatische Versorgung. In an sich bekannter Weise ist an diesen Druckluftanschluss 4 über eine pneumatische Versorgungsleitung ein von einer Ansteuereinheit gesteuertes Ventil, insbesondere Magnetventil, angeschlossen, das in einem gleich bleibenden iterativen Takt zwischen etwa 1 Hz und 50 Hz Druckluftpulse über den Druckluftanschluss einkoppelt. Das Ventil ist nicht gezeigt und kann auch in dem dargestellten Gerät selbst eingebaut sein.

Das Gerät ist im Übrigen als mit der Hand einer Bedienungsperson zu haltendes Gerät ausgebildet, das über die erwähnte Pneumatikleitung an eine nicht gezeigte Basisstation mit der Ansteuereinheit und dem Kompressor angeschlossen ist und auf den Patienten manuell aufgesetzt werden kann. Es dient zur Behandlung von Weichgewebe, insbesondere Muskeln und Faszien.

In dem Gehäuse ist über einen Einsatz 5 ein Führungsrohr 6 gehalten, dessen bei der Anwendung körperfernes Ende in der Zuluftkappe 2 endet und dort mit dem Druckluftanschluss 4 kommuniziert. Das in der Anwendung körperseitige Ende des Führungsrohres 6 endet in einem Teil des Einsatzes 5, der in die Applikatorkappe 3 hineinragt, und zwar kurz vor dem dortigen Ende des Einsatzes 5 und einem Innenraum 7 in der Applikatorkappe 3.

In dem Innenraum 7, der in eine in der Anwendung körperseitige Applikatoröffnung übergeht, ist ein erster und in Fig. 1 schraffierter Teil eines Applikator 9 aufgenommen. Dieser stützt sich über ein elastisches Schlauchelement 10 aus einem Elastomer an einer radialen Schulter ab. Ein zur körperfernen Seite gerichtetes und die Aufprallfläche beinhaltendes Ende 15 des Applikators 9 stützt sich über einen O-Ring 12 an dem Einsatz 5 ab, und zwar an einer das bereits erwähnte Ende des Einsatzes 5 umgebenden Stirnfläche. Dabei liegt der O-Ring 12 zwischen dieser Stirnfläche und einer Schulter des Applikators 9. Die Applikatoröffnung dient dabei zu einer in der Längsrichtung verschiebbaren Führung des Applikators 9 und fixiert diesen quer zur Längsrichtung. Die Axialverschiebbarkeit ist nur durch die Nachgiebigkeit des Elastomerelements 10 begrenzt und kann bei einem in Luft betriebenen Gerät relativ zum Restgerät auch deutlich über 1 mm liegen.

Der Applikator 9 weist als zweiten Teil das nicht schraffierte Element 11 auf, das den eigentlichen auf die Haut aufzusetzenden Applikator bildet. Der Applikator 9 ist durch Abschrauben der Applikatorkappe 3 austauschbar.

In dem angrenzenden Bereich des Führungsrohres 6 ist ein in Figur 1 mit dem Applikator 9 in Kontakt stehendes Projektil 13 eingesetzt. Dieses passt (in Bezug auf das Führungsrohr und die im Wesentlichen zylindrische Geometrie des Projektils 13) radial mit geringem Spiel hinein. Das Projektil 13 kann durch Druckunterschiede der Luftsäule in dem Führungsrohr 6 vor und hinter ihm (d. h. in Figur 1 rechts und links des Projektils 13) in dem Führungsrohr hin- und herbewegt werden und insbesondere auf den Applikator 9 zu beschleunigt werden. Hierzu wird es aus einer Ausgangsposition (nicht gezeigt) in Figur 1 links durch einen Druckluftstoß durch den Druckluftanschluss 4 beschleunigt und trifft mit seiner dem Applikator 9 zugewandten Frontfläche auf den Applikator 9 auf, und zwar auf eine körperabgewandte Prallfläche 15 davon.

Die Rückbewegung des Projektils 13 erfolgt zusätzlich zu einem Zurückprallen nach der Kollision durch ein Rückströmen der Luft aus einer das Führungsrohr 6 innerhalb des Einsatzes 5 umgebenden Staukammer 14. In diese wird die Luft bei der Beschleunigung des Projektils 13 in Richtung zu dem Prallkörper 9 verdrängt und damit dort komprimiert. Wenn das Magnetventil den Druck wegschaltet und gleichzeitig den Raum hinter dem Projektil entlüftet, wird das Projektil 13 damit in die Ausgangsstellung zurückbewegt. Dies kann natürlich auch durch eine zusätzliche oder alternative Druckbeaufschlagung der Staukammer 14 oder eines anderen Luftvolumens körperseitig von dem Projektil 13 erfolgen. Das in der Anwendung körperferne Ende des Führungsrohres 6 endet in einem Magnethalter für das Projektil 13.

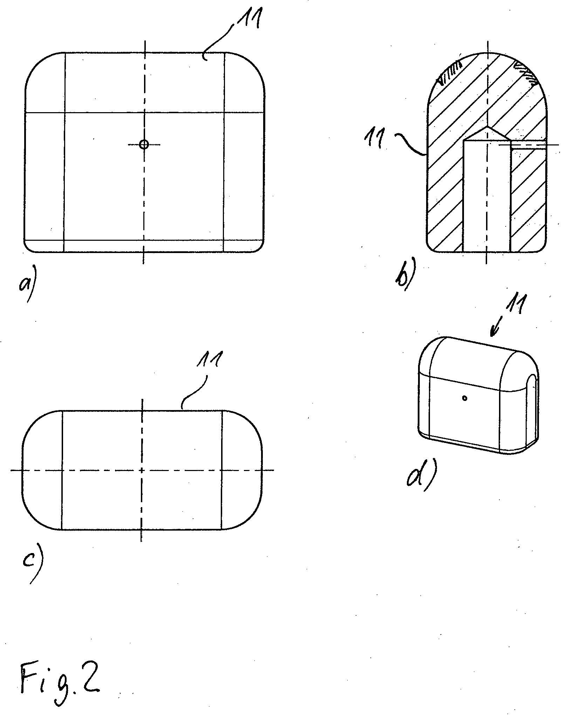

In Figur 1 ist der Applikator 9 im Wesentlichen zu seinem in Bezug auf die Längsachse des Geräts radial schlankeren Teil 16 schraffiert gezeichnet und im übrigen Teil 11 schraffurfrei. Wenn man sich den schraffierten Teil 16 mit einer zum Beispiel leicht konvexen Begrenzungslinie abgeschlossen vorstellt, würde er eine konventionelle Applikatorform bilden. In dieser Form würde sich der Applikator 9 vor allem für das vertikale Aufsetzen auf die Körperoberfläche des Patienten eignen. Erfindungsgemäß ist der Applikator 9 nun in Richtung zu dem Patientenkörper vergrößert, und zwar um den nicht schraffierten Teil 11. Dieser hat in einer Richtung senkrecht zur Längsrichtung gesehen die in Figur 1 angegebene Form und in der zweiten Richtung senkrecht zur ersten die in Figur 2b dargestellte Form. Dabei ist der Applikator 9 in Figur 1 ungefähr im richtigen Größenverhältnis zum Restgerät dargestellt, in Figur 2 aber vergrößert. Seine in Bezug auf Figur 1 vertikale Erstreckung beträgt hier beispielhaft 30 mm bei einer Länge (in Längsrichtung) von 25 mm und patientenkörperseitigen Krümmungsradien im Übergang zwischen den beiden Begrenzungsflächen in der Projektion gemäß Figur 1 von 5 mm. In Figur 2b hingegen erkennt man bei gleicher Länge (in Längsrichtung) von 25 mm eine schmalere Erstreckung senkrecht dazu, nämlich von 15 mm, und patientenkörperseitig in der Projektion der Figur 2b eine halbkreisförmige Verrundung mit einem Radius von 7,5 mm.

In der dritten Darstellung c der Figur 2 sieht man auf den vorderen Teil 11 des Applikators 9 in Längsrichtung und erkennt eine rechteckige Grundform (mit den bereits erwähnten Kantenlängen von 30 mm bzw. 50 mm), aber mit abgerundeten Ecken mit einem Krümmungsradius von jeweils 5 mm. Die vierte Darstellung d veranschaulicht die Applikatorform in perspektivischer Ansicht.

Die Frontfläche des vorderen Teils 11 des Applikators 9 ist also in der Perspektive der Darstellung in Figur 2b halbzylinderförmig, aber mit in zwei Projektionsebenen abgerundeten Ecken mit Krümmungsradien von 5 mm, vgl. die Figuren. Diese verrundete Halbzylinderform hat schon ohne Berücksichtigung der zusätzlichen Verrundungen einen Anteil von einem Drittel mit Winkeln der jeweiligen lokalen Flächenanteile (bzw. der Normalen dazu) zur Längsrichtung zwischen 30° und 60°; unter Berücksichtigung der weiteren Verrundungen liegt der Anteil noch etwas höher. Die entsprechenden Winkelsektoren und Flächenbereiche sind durch Radien und Schraffuren angedeutet. Figur 2b zeigt bereits, dass der schräge Frontflächenanteil in den festgelegten Winkelbereich zwischen 30° und 60° ein Drittel der hier im Schnitt halbkreisförmigen Frontfläche ausmacht. Da die Verrundungen in der Frontansicht der Figur 2c dazu führen, dass es auch seitlich (in Figur 2c nach links und nach rechts) einen entsprechenden schrägen Frontflächenanteil gibt, liegt der relative Anteil insgesamt bei etwa einem Drittel und damit über 30 %.

Er ist im Hinblick auf die Längsrichtung von einer zweizähligen Symmetrie und kann mit den beiden jeweils 30 mm breiten, einem Viertel-Zylinder entsprechenden Frontflächenanteilen auf die Körperoberfläche des Patienten aufgelegt werden. Wenn dabei angenähert ein Aufliegewinkel von 45° verwendet wird, dann sind die Flächenanteile mit einem Winkel zwischen 30° und 60° zur Längsrichtung besonders bedeutsam. Der Relativanteil dieser Flächenanteile in Verbindung mit der im Vergleich zum Stand der Technik besonders breiten Ausführung (30 mm) und der starken Verrundung lassen die Applikatorfrontfläche besonders geeignet erscheinen, um auf der Körperoberfläche streichend oder schiebend bewegt zu werden. Aus den gleichen Gründen können Stöße mit relativ großen Hubwerten von über 1 mm vergleichsweise schonend und großflächig in den Körper eingekoppelt werden.

Der Applikator 9 hat insgesamt und insbesondere mit seinem vorderen Teil 11 eine bezüglich der Längsrichtung zweizählige Symmetrie (und zusätzlich zwei Spiegelsymmetrien, wobei sich die Symmetrieebenen in der Längsrichtung schneiden) und ist dabei in seiner gesamten Frontfläche konvex. Alle auftretenden Krümmungsradien liegen bei mindestens 5 mm und die schräge Frontfläche liegt bei ungefähr über 2,5 cm² insgesamt. Da bei einer typischen Anwendung nur eine Breitseite des Applikators angesetzt wird, also zum Beispiel in Figur 2b der nach links oben weisende Teil der Frontfläche, steht hiervon nur ein Teil im Hautkontakt. Wenn man die seitlichen abgerundeten Bereiche (in Figur 2c rechts und links), die nicht auf der Haut aufliegen, berücksichtigt, dürfte die Kontaktfläche in der Größenordnung von 1,0 bis 1,2 cm² liegen.

Der schräge Frontflächenanteil konzentriert sich in Blickrichtung der Längsrichtung auf die beiden langen Seiten des abgerundeten Rechtecks.

Der Applikator 9 kann z. B. aus Aluminium, Titan, Kunststoff, Keramik oder Holz bestehen.

Hinsichtlich seiner Ausbaubarkeit bietet es sich an, seine Bestandteile 11 und 16 fest miteinander zu verbinden, zum Beispiel durch eine Steck- oder Schraubverbindung. Die Bestandteile 11 und 16 können aus unterschiedlichen Materialien hergestellt sein.

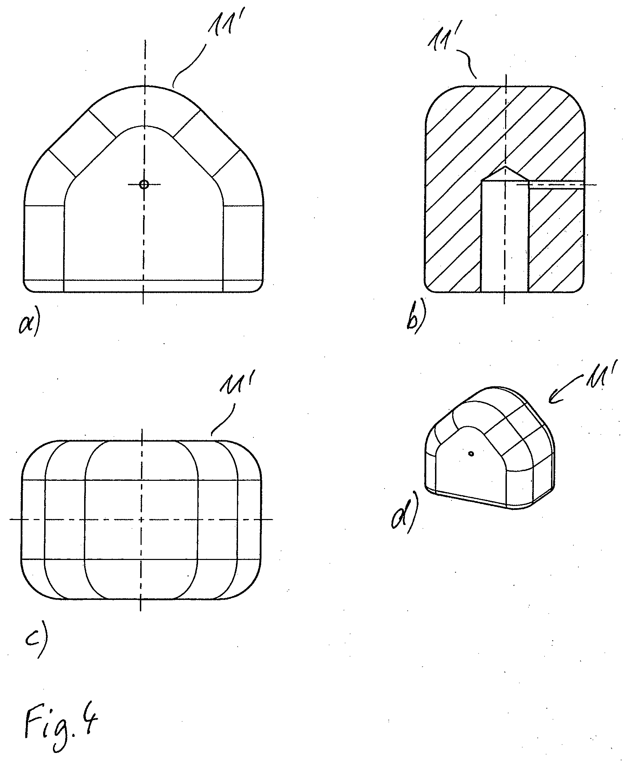

Alternativ könnte der vordere Teil 11 des Applikators auch an einem Applikator ähnlich der Darstellung in Figur 4 der bereits zitierten US 2011/0054367 A1 vorgesehen sein und auch hier näherungsweise mit seiner breitesten Ausdehnung dem Gerätedurchmesser entsprechen. Dann könnte er nach vorn durch die Öffnung in der Applikatorkappe abgezogen werden, nachdem diese gelöst und andere Teile abgebaut worden sind.

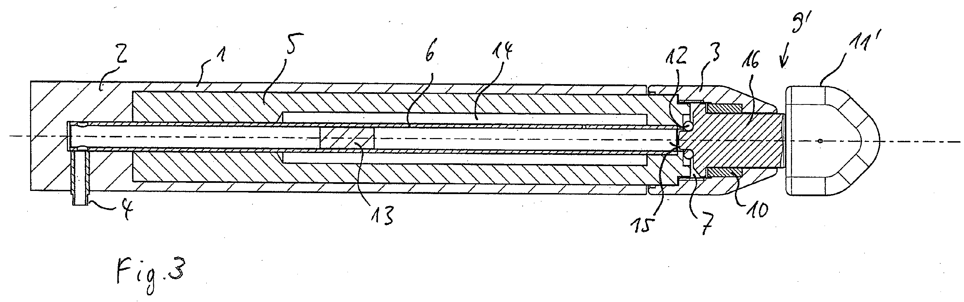

Eine alternative zweite Ausführungsform zeigen die Figuren 3 und 4a bis d, wobei in letzteren lediglich der vordere Applikatorteil 11' dargestellt ist, den Fig. 3 in gleicher Weise an einem Gerät zeigt, wie Fig. 1 oder den man sich wie in dem zitierten US-Dokument an einem Gerät vorstellen kann. Einzeldarstellungen der Fig. 4 entsprechen abgesehen von der veränderten Applikatorform den Einzeldarstellungen der Fig. 2, wobei die Breite des Applikators 11' gemäß Fig. 4b 20 mm (statt 15 mm in Fig. 2b) beträgt.

Man erkennt in Figur 4a relativ große schräge Frontflächenanteile in einem Winkel von 45° zur Längsrichtung, wobei sich diese Anteile auch in die mit einem Radius von 10 mm verrundeten Übergänge zwischen den Schrägflächen untereinander und zwischen den Schrägflächen und den zur Längsrichtung des Geräts parallelen Flächen hineinerstrecken. In der Fig. 4b fehlt die halbzylindrische Form der Fig. 2b; stattdessen handelt es sich hier um ein Rechteck mit Krümmungsradien von 5 mm den Ecken oben. Die schrägen Frontflächenanteile bestehen also im Wesentlichen in den planen und unter 45° zur Längsrichtung stehenden Flächen in Figur 4 mit einem kleinen Teil der angrenzenden Verrundung, wie schraffiert angedeutet. Diese machen genähert eine Fläche von gut 2 cm² aus, wobei sich die schrägen Frontflächenanteile (bzgl. der Perspektive in Längsrichtung) auf einen Winkelbereich von ganz ungefähr 140° beschränken. Im Übrigen gelten die Aussagen zum ersten Ausführungsbeispiel. Bevorzugtes Material in beiden Fällen ist der Kunststoff POM (ein hochmolekularer Thermoplast, gelegentlich als Acetal bezeichnet).

## Patentansprüche

1. Gerät zur Behandlung des menschlichen oder tierischen Körpers mit
- einem Applikator (9,11,11',16) zum Auflegen auf den Körper von außen,
- einem Gehäuse (1-3), in dem der Applikator (9,11,11',16) gehalten ist, und
- einem Mechanismus (4,6,13) zum Erzeugen von Stößen des Applikators (9,11,11',16) relativ zu dem Gehäuse (1-3) in einer Stoßrichtung, so dass die Stöße bei dem Auflegen in den Körper eingekoppelt werden können,
wobei der Applikator (9,11,11',16)
einen schrägen Frontflächenanteil hat,
der zumindest 30 % einer in Bezug auf die Stoßrichtung vorderen Frontfläche des Applikators (9,11,11',16) beträgt,
der in Bezug auf die Stoßrichtung schräg nach außen weist und der dabei einen Winkel zu der Stoßrichtung zwischen 30° und 60° bildet,
**dadurch gekennzeichnet, dass** der schräge Frontflächenanteil in dem Sinn kantenfrei ist, dass alle auftretenden Krümmungsradien mindestens 2 mm betragen.

2. Gerät nach Anspruch 1, bei dem der Mechanismus (4,6,13) zum Erzeugen der Stöße ein Projektil (13) und eine Einrichtung (4, 13) zum Beschleunigen des Projektils in solcher Weise aufweist, dass das Projektil (13) auf den Applikator (9,11,11',16) schlägt und dadurch den Stoß erzeugt, vorzugsweise eine pneumatische Einrichtung zur Beschleunigung des Projektils (13).

3. Gerät nach einem der vorstehenden Ansprüche, bei dem der schräge Frontflächenanteil flach oder konvex, aber nicht konkav ist.

4. Gerät nach einem der vorstehenden Ansprüche, bei dem die Frontfläche insgesamt in dem Sinn kantenfrei ist, dass alle auftretenden Krümmungsradien mindestens 2 mm betragen.

5. Gerät nach einem der vorstehenden Ansprüche, bei dem der schräge Frontflächenanteil insgesamt mindestens 1,5 cm² beträgt.

6. Gerät nach einem der vorstehenden Ansprüche, bei dem der schräge Frontflächenanteil einen Anteil von mindestens 80 % mit einem Krümmungsradius von mindestens 5 mm aufweist.

7. Gerät nach einem der vorstehenden Ansprüche, bei dem der schräge Frontflächenanteil höchstens vierfach, vorzugsweise dreifach oder sogar höchstens zweifach rotationssymmetrisch zur Stoßrichtung ist.

8. Gerät nach Anspruch 7, bei dem sich der schräge Frontflächenanteil auf höchstens vier Seiten beschränkt, vorzugsweise auf höchstens drei oder sogar höchstens zwei Seiten.

9. Gerät nach einem der vorstehenden Ansprüche, bei dem der Applikator in seinem zum Auflegen ausgelegten Teil besteht aus Metall, insbesondere Aluminium oder Titan, Kunststoff, Keramik oder Holz.

10. Gerät nach einem der vorstehenden Ansprüche, das ausgelegt ist für einen Hub des Stoßes in der Stoßrichtung relativ zu dem Gehäuse von mindestens 1 mm.

## Claims

1. An apparatus for treating a human or animal body, having
- an applicator (9,11,11',16) for being placed on said body from the outside,
- a housing (1-3) in which said applicator (9,11,11',16) is held, and
- a mechanism (4,6,13) for generating strokes of said applicator (9,11,11',16) relative to said housing (1-3) in a stroke direction so that said strokes can be coupled into said body when said applicator is placed on said body,
**characterized in that** said applicator (9,11,11',16)
has an oblique front area portion
which amounts to at least 30 % of a front area of said applicator (9,11,11',16), lying in front with respect to said stroke direction,
said front area portion pointing outwards obliquely with respect to said stroke direction,
and said front area portion having an angle to said stroke direction between 30° and 60°.

2. The apparatus according to claim 1, wherein said mechanism (4,6,13) for generating said strokes comprises a projectile (13) and a device (4,13) for accelerating said projectile in such a way that said projectile hits said applicator (9,11,11',16) and generates said stroke, thus, preferably a pneumatic device for accelerating said projectile (13).

3. The apparatus according to one of the preceding claims, wherein said oblique front area portion is plane or convex but is not concave.

4. The apparatus according to one of the preceding claims, wherein said front area as a whole is free of edges insofar as all radii of curvature shall be 2 mm at minimum.

5. The apparatus according to one of the preceding claims, wherein said oblique front area portion has an overall area of 1.5 cm² at minimum.

6. The apparatus according to one of the preceding claims, wherein said oblique front area portion comprises a portion of 80 % at minimum, in which a radius of curvature is 5 mm at minimum.

7. The apparatus according to one of the preceding claims, wherein said oblique front area portion has a rotational symmetry with respect to said stroke direction, which is fourfold at maximum, preferably threefold or even twofold at maximum.

8. The apparatus according to claim 7, wherein said oblique front area portion is formed on four sides at a maximum, preferably on three sides at maximum or even two sides at maximum.

9. The apparatus according to one of the preceding claims, wherein a part of said applicator, which is adapted for being placed on said body, is made of metal, in particular aluminium or titanium, synthetic material, ceramics or wood.

10. The apparatus according to one of the preceding claims, which is adapted for a travel of said stroke of 1 mm at minimum in said stroke direction relatively to that housing.

## Revendications

1. Appareil pour le traitement du corps humain ou animal, comportant :
- un applicateur (9, 11, 11', 16) destiné à une application sur ledit corps depuis l'extérieur,
- un boîtier (1-3) dans lequel l'applicateur (9, 11, 11', 16) est retenu, et
- un mécanisme (4, 6, 13) permettant à l'applicateur (9, 11, 11', 16) d'exercer des coups par rapport au boîtier (1-3) dans une direction d'impact, de manière que les coups peuvent être répercutés dans le corps suite à ladite application,
l'applicateur (9, 11, 11', 16) possédant une partie de surface frontale en biais,
ladite partie représentant au moins 30 % d'une surface frontale de l'applicateur (9, 11, 11', 16), vue dans la direction d'impact,
ladite partie étant orientée vers l'extérieur en biais, vue dans la direction d'impact, et
ladite partie formant un angle compris entre 30° et 60° par rapport à la direction d'impact,
**caractérisé en ce que** la partie de surface frontale en biais est exempte d'arêtes en ce sens que tous les rayons de courbure en présence font au moins 2 mm.

2. Appareil selon la revendication 1, dans lequel le mécanisme (4, 6, 13) permettant d'exercer des coups présente un projectile (13) et un dispositif (4, 13) permettant une accélération du projectile de telle manière que le projectile (13) frappe l'applicateur (9, 11, 11', 16) en produisant ainsi un coup, s'agissant de préférence d'un dispositif pneumatique d'accélération du projectile (13).

3. Appareil selon l'une des revendications précédentes, dans lequel la partie de surface frontale en biais est plate ou convexe mais nullement concave.

4. Appareil selon l'une des revendications précédentes, dans lequel la surface frontale dans son ensemble est exempte d'arêtes en ce sens que tous les rayons de courbure en présence font au moins 2 mm.

5. Appareil selon l'une des revendications précédentes, dans lequel la partie de surface frontale en biais représente dans son ensemble au moins 1,5 cm².

6. Appareil selon l'une des revendications précédentes, dans lequel la partie de surface frontale en biais présente une partie représentant au moins 80 % dont le rayon de courbure est d'au moins 5 mm.

7. Appareil selon l'une des revendications précédentes, dans lequel la partie de surface frontale en biais présente au maximum une quadruple, de préférence triple voire au maximum double symétrie en rotation par rapport à la direction d'impact.

8. Appareil selon la revendication 7, dans lequel la partie de surface frontale en biais se limite au maximum à quatre faces, de préférence au maximum à trois faces, voire au maximum à deux faces.

9. Appareil selon l'une des revendications précédentes, dans lequel l'applicateur, dans sa partie conçue pour l'application sur le corps, se compose de métal, notamment d'aluminium ou de titane, de plastique, de céramique ou de bois.

10. Appareil selon l'une des revendications précédentes, conçu pour une course d'impact, dans la direction d'impact par rapport au boîtier, qui est d'au moins 1 mm.
